# EUROPÄISCHE PATENTANMELDUNG

(11) **EP 0 997 117 A2**
(43) Veröffentlichungstag der Anmeldung: **03.05.2000**
(21) Anmeldenummer: 99118751.9
(22) Anmeldetag: 23.09.1999
(51) Int. Cl.: A61F 2/34

(54) **Hüftgelenkpfanne**

(30) Priorität: 24.09.1998 DE 19843797
(71) Anmelder: GMT Gesellschaft für medizinische Technik mbH, D-22767 Hamburg (DE); Waldemar Link (GmbH & Co.), 22339 Hamburg (DE)
(72) Erfinder: Otto, K. B. Dr., 25474 Ellerbek (DE); Keller, Arnold, 23863 Kayhude (DE)
(74) Vertreter: Heldt, Gert, Dr. Dipl.-Ing.

(57) **Zusammenfassung**

Eine Hüftgelenkpfanne mit einer in einer Höhlung eines sie aufnehmenden Beckenknochens anliegenden Außenfläche und einer einen Gelenkkopf eines künstlichen Hüftgelenkes aufnehmenden Aufnahmefläche ist von einem sie begrenzenden Aufnahmerand umgeben. Der Aufnahmerand weist an mindenstens einer Stelle eine den von der Aufnahmefläche aufgenommenen Gelenkkopf teilweise umfassende Erhöhung auf. Diese ist an einer Stelle vorgesehen, an der sie den Gelenkkopf in seiner Bewegungsfreiheit am wenigstens behindert, und weist an ihrem vom Aufnahmerand am weitesten aufragenden Scheitelpunkt einen Abstand von einem tiefsten Punkt der Aufnahmefläche auf, die den Gelenkkopf weitgehend führend umfaßt.

## Beschreibung

Die Erfindung betrifft eine Hüftgelenkpfanne mit einer in einer Höhlung eines sie aufnehmenden Beckenknochens anliegenden Außenfläche und einer einen Gelenkkörper eines künstlichen Hüftgelenkes aufnehmenden Aufnahmefläche, die von einem sie begrenzenden Aufnahmerand umgeben ist.

Eine derartige Hüftgelenkpfanne ist beispielsweise aus der US 4,883,490 und der US 4,123,806 bekannt. Nachteilig bei den bekannten Hüftgelenkpfannen ist, daß es bei bestimmten Bewegungen eines mit ihr versehenen Beines bzw. Femurs zu einer unerwünschten Luxation kommen kann. Um die Gefahr des Herausspringens des Gelenkkopfes aus der Hüftgelenkpfanne zu verringern, könnte die Tiefe des Kugelabschnittes in der Hüftgelenkpfanne vergrößert werden. Dies würde aber andererseits gleichzeitig zu einer Bewegungseinschränkung und damit zu einer unerwünschten Funktionseinschränkung der Hüftgelenkpfanne bzw. der gesamten Hüftgelenksendoprothese führen.

Aufgabe der vorliegenden Erfindung ist es daher, die bekannten Hüftgelenkpfannen so zu verbessern, daß einerseits das Risiko einer unerwünschten Luxation verringert wird und anderseits eine unerwünschte Bewegungseinschränkung weitgehend vermieden wird.

Diese Aufgabe wird erfindungsgemäß dadurch gelöst, daß der Aufnahmerand an mindestens einer Stelle eine den von der Aufnahmefläche aufgenommenen Gelenkkopf teilweise umfassende Erhöhung aufweist.

Dadurch, daß der Aufnahmerand eine Erhöhung aufweist, ist es möglich, in diesem Bereich eine Tiefe der Hüftgelenkpfanne entstehen zu lassen, die größer als der Radius des Kugelabschnittes ist, so daß ein eingesetzter Gelenkkopf von der Aufnahmefläche der Hüftgelenkpfanne formschlüssig umfaßt wird bzw. in der Hüftgelenkpfanne einrastet. Im gefährdeten Bewegungsbereich findet somit eine beabsichtigte Blockierung statt, die eine Luxation verhindert. Im nicht von der Erhöhung betroffenen Bereich kommt es jedoch nicht zu einer unnötigen Blockierung oder unerwünschten Bewegungseinschränkung.

Gemäß einer bevorzugten Ausführungsform der Erfindung ist die Erhöhung an einer Stelle vorgesehen, an der sie den Gelenkkopf in seiner Bewegungsfreiheit am geringsten behindert. Auf diese Weise wird erreicht, daß einerseits Luxationen stark eingeschränkt werden können und andererseits die Bewegungsfreiheit des Gelenkkopfes nur in solchen Bereichen behindert wird, in denen nur sehr selten Bewegungsvorgänge stattfinden.

Gemäß einer weiteren bevorzugten Ausführungsform der Erfindung weist die Erhöhung an ihrem vom Aufnahmerand am weitesten aufragenden Scheitel einen Abstand von einem tiefsten Punkt der Aufnahmefläche auf, der den Gelenkkopf weitgehend führend umfaßt. Auf diese Weise wird erreicht, daß der Gelenkkopf in die Aufnahmefläche einrasten kann, so daß er innerhalb der Aufnahmefläche Bewegungen ausführen kann, die lediglich durch die Erhöhung beeinträchtigt werden, ansonsten jedoch den wesentlichen Teil der für einen Femur gewünschten Bewegungsfreiheit eröffnet.

Gemäß einer weiteren bevorzugten Ausführungsform der Erfindung ragt die Erhöhung vom tiefsten Punkt der Aufnahmefläche zum Scheitelpunkt in einer Richtung auf, in der ein das künstliche Hüftgelenk aufnehmender Femur gegenüber einem gestrecktem Standbein schräg nach hinten verläuft und dabei eine vom Standbein in einer Geradeausrichtung aufgespannte gedachte Bewegungsebene in einem spitzen Winkel schneidet. Eine derartige Bewegungsrichtung ist für einen Femur unüblich und wird auch nur selten ausgeführt. Eine Behinderung im Bezug auf diese Bewegungsrichtung macht sich daher für einen Träger eines Hüftgelenkes so wenig bemerkbar, daß er kaum beeinträchtigt wird. Trotzdem besitzt die Erhöhung den entscheidenen Vorteil, daß sie in der Lage ist, Luxationen des Hüftgelenkes weitgehend zu verhindern.

Gemäß einer weiteren bevorzugten Ausführungsform der Erfindung ist die Aufnahmefläche als ein Kugelabschnitt einer von einer Hohlkugel umschlossenen Innenfläche ausgebildet, von der eine dem als Kugelkopf ausgebildeten Gelenkkopf zugewendete Gleitfläche der Erhöhung einen Teil bildet. Diese an der Erhöhung ausgebildete Gleitfläche fügt sich in die als Hohlkugel ausgebildete Aufnahmefläche ein, so daß sie für einen als Kugelkopf ausgebildeten Gelenkkopf optimale Führungsverhältnisse ermöglicht.

Gemäß einer weiteren bevorzugten Ausführungsform der Erfindung ist der Aufnahmerand in einem ersten Teilbereich als Randbereich eines etwa in horinzontaler Ebene verlaufenden Kugelschnittes ausgebildet, auf dessen Ebene eine durch den tiefsten Punkt verlaufende Pfannenachse senkrecht steht. Einem ersten Teilbereich des Aufnahmerandes schließt sich ein zweiter, die Erhöhung aufweisender Teilbereich an, der von einem im ersten Teilbereich benachbarten Anlaufende des zweiten Teilbereichs bis zum Scheitelpunkt der Erhöhung hin ansteigt. Durch das Ansteigen der Erhöhung im zweiten Teilbereich kann ein Optimum zwischen Luxationsrisiko einerseits und Bewegungsfreiheit andererseits erzielt werden.

Nach einer weiteren bevorzugten Ausführungsform der Erfindung ist zwischen einem dem Anlaufende des zweiten Teilbereichs abgewandten ersten Ende des ersten Teilbereichs und dem Scheitelpunkt der Erhöhung des zweiten Teilbereichs ein dritter Teilbereich angeordnet, in dem der Aufnahmerand in Richtung auf den tiefsten Punkt zur Freigabe eines Schwenkraumes abgesenkt ist, innerhalb dessen ein im Femur befestigbarer Schaft des Kugelkopfes schwenkbar lagerbar ist. Dieser Schwenkraum dient dazu, daß der mit dem Gelenkkopf verbundene Schaft bei der Ausführung von Schwenkbewegungen von dem Aufnahmerand nicht beeinträchtigt wird. Die im Bereich des Schwenkraumes verminderte Führung des Gelenkkopfes wird durch die Erhöhung ausreichend kompensiert. Aus diesem Grunde fällt der Schwenkraum unmittelbar in Richtung auf das erste Ende vom Scheitelpunkt ab. Außerdem ist der Aufnahmerand im Bereich des Schwenkraumes in einer Ebene angeschrägt, die im spitzen Winkel zu einer sich durch die Pfannenachse erstreckenden lotrechten Ebene verläuft. Diese vom Schwenkraum aufgespannte Ebene ist so angelegt, daß in ihrem Bereich eine unbehelligte Bewegung des am Gelenkkopf befestigten Schaftes möglich ist.

Gemäß einer weiteren bevorzugten Ausführungsform der Erfindung weist der Aufnahmerand im Schwenkraum eine den Kugelkopf formschlüssig führende Höhe gegenüber dem tiefsten Punkt und eine das Einsetzen des Kugelkopfes in die Aufnahmefläche erlaubende gebrochene Kante auf. Auf diese Weise kann der Aufnahmerand im Bereich des Schwenkraumes relativ hoch an dem Gelenkkopf herangezogen werden, ohne daß das Einsetzen des Gelenkkopfes erschwert wird. Dieser wird über die gebrochene Kante in die Aufnahmefläche eingeführt.

Gemäß einer bevorzugten Ausführungsform der Erfindung ist der Aufnahmerand in einem ersten Teilbereich als Rand eines etwa quer zu einer Pfannenachse verlaufenden Kugelschnittes ausgebildet. An den ersten Teilbereich des Aufnahmerandes schließt ein zweiter, die Erhöhung aufweisender Teilbereich an, der von einem dem ersten Teilbereich benachbarten Anlaufende des zweiten Teilbereiches bis zu einem Scheitelpunkt der Erhöhung hin ansteigt. Durch das Ansteigen der Erhöhung in dem zweiten Teilbereich kann ein Optimum zwischen Luxationsrisiko und Bewegungsfreiheit erzielt werden.

Nach einer weiteren bevorzugten Ausführungsform der Erfindung ist zwischen einem dem Anlaufende des zweiten Teilbereiches abgewandten ersten Ende des ersten Teilbereiches und dem Scheitelpunkt der Erhöhung des zweiten Teilbereiches ein dritter Teilbereich angeordnet, der den Rand eines in einem spitzen Winkel zur Pfannenachse angeordneten Kugelschnittes bildet, so daß in diesem Bereich eine Tiefe entsteht, die geringer ist als die Tiefe des ersten Teilbereiches und kleiner als der Radius des Kugelabschnittes. Durch den Kugelschnitt im weniger gefährdeten dritten Teilbereich wird durch die verminderte Tiefe eine erhöhte Bewegungsfreiheit erzielt, ohne das Luxationsrisiko gegenüber dem bekannten Stand der Technik zu erhöhen.

Nach einer weiteren bevorzugten Ausführungsform der Erfindung weist die Außenfläche eine Mehrzahl von etwa konzentrisch zur Pfannenachse angeordneten Abstandshaltern auf. Beim Einzementieren der Hüftgelenkpfanne wird durch die Abstandshalter eine gleichmäßigere Schichtdicke des zu verwendenden Knochenzementes erreicht.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

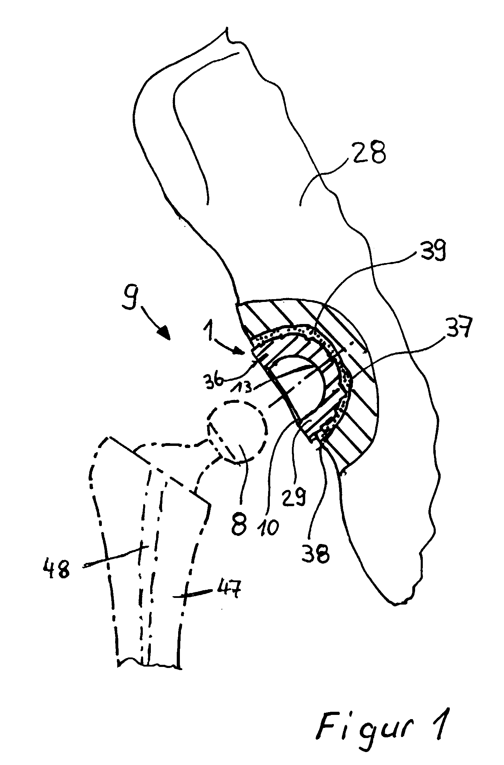

- Figur 1:: Eine schematische teilweise geschnittene Vorderansicht einer in einem Beckenknochen fixierten Hüftgelenkpfanne und einen Oberschenkelknochen mit einem Kugelkopf eines künstlichen Hüftgelenkes,

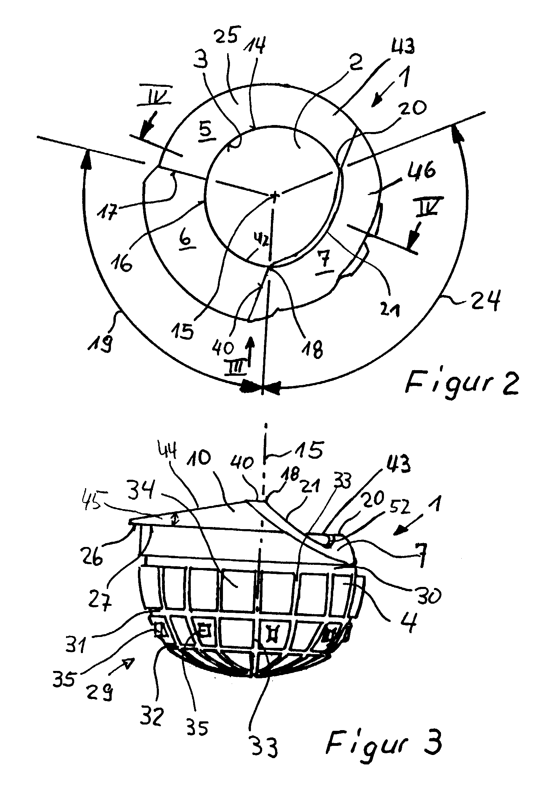

- Figur 2:: eine Draufsicht auf eine Hüftgelenkpfanne,

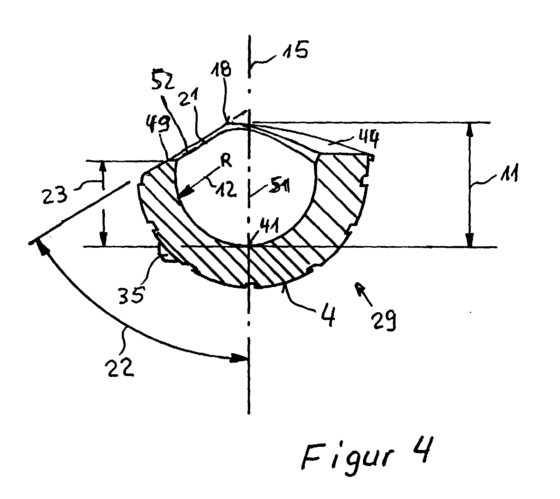

- Figur 3:: eine Seitenansicht auf die Hüftgelenkpfanne von Figur 2 aus Richtung III,
- Figur 4:: eine Seitenansicht der Hüftgelenkpfanne von Figur 2 entlang der Linie IV-IV geschnitten und

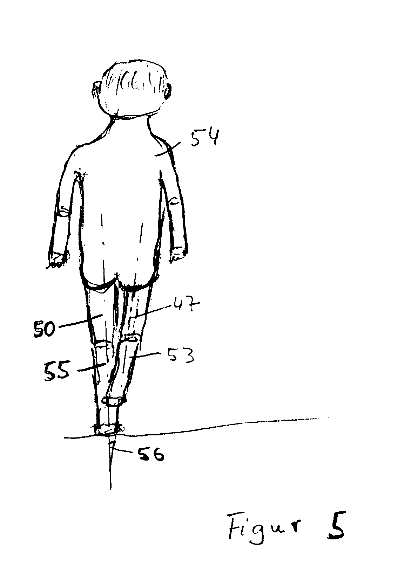

- Figur 5:: eine Rückansicht einer menschlichen Figur mit einem schräg nach hinten angewinkelten rechten Bein.

Eine Hüftgelenkpfanne (1) besteht im wesentlichen aus einer Aufnahmefläche (2) mit einem Aufnahmerand (3) und aus einer Außenfläche (4), die von Teilflächen (5,6,7) begrenzt wird.

Die Aufnahmefläche (2) ist konkav ausgeformt und kann einen Kugelabschnitt zur Aufnahme eines Gelenkkopfes (8) bilden, der mit einer Hüftgelenkpfanne (1) ein künstliches Hüftgelenk (9) darstellt. Der Gelenkkopf (8) kann als Kugelkopf ausgebildet sein, an dem ein Schaft (48) befestigt ist, der in einem Femur (47) verankert werden kann. Dieser erstreckt sich beispielsweise durch ein rechtes Bein (53) eines Menschen (54).

Die Aufnahmefläche (2) wird von dem Aufnahmerand (3) begrenzt. Die Aufnahmefläche (2) weist an dem Aufnahmerand (3) eine Erhöhung (10) auf, so daß in diesem Bereich ein Abstand (11) zwischen einem die Erhöhung (10) begrenzenden Scheitelpunkt (18) und einem tiefsten Punkt (41) der Aufnahmefläche entsteht, der größer als ein Radius (12) eines von der Aufnahmefläche (2) gebildeten Kugelabschnittes (13) ist.

Der Aufnahmerand (3) ist in einem ersten Teilbereich (14) als ein etwa in einer horizontalen Ebene (43) verlaufender Rand ausgebildet. Auf dieser horizontalen Ebene (43) steht eine sich durch die Aufnahmefläche (2) erstreckende Pfannenachse (15) etwa senkrecht. Die horizontale Ebene (43) begrenzt einen von der Aufnahmefläche (2) gebildeten Kugelschnitt.

An den ersten Teilbereich (14) des Aufnahmerandes (3) schließt sich ein zweiter, die Erhöhung (10) aufweisender Teilbereich (16) an, der von einem dem ersten Teilbereich (14) benachbarten Anlaufende (17) des zweiten Teilbereiches (16) bis zu einem Scheitelpunkt (18) bzw. einer Scheitellinie (40) der Erhöhung (10) hin ansteigt. Der zweite Teilbereich (16) spannt zwischen seinem Anlaufende (17) und dem Scheitelpunkt (18) einen zweiten Sektorwinkel (19) auf. Der zweite Sektorwinkel (19) beträgt etwa 110°. Zwischen einem dem Anlaufende (17) des zweiten Teilbereiches (16) abgewandten ersten Ende (20) des ersten Teilbereiches (14) und dem Scheitelpunkt (18) bzw. der Scheitellinie (40) der Erhöhung (10) des zweiten Teilbereiches (16) ist ein dritter Teilbereich (21) angeordnet. Der dritte Teilbereich (21) besitzt einen in einer schrägen Ebene (49) geschnittenen Rand. Diese schräge Ebene (49) verläuft in einem spitzen Winkel (22) zu einer sich durch die Pfannenachse (15) erstreckenden lotrechten Ebene (51). Der dritte Teilbereich (21) bildet einen Schwenkraum (46), in dem der im Femur (47) verankerte Schaft (48) des Gelenkkopfes (8) Schwenkbewegungen beispielsweise bei Gehbewegungen des Menschen (54) ausführen kann.

Aus diesem Grunde ist der im Bereich der dritten Teilfläche (7) angeschrägte Rand relativ weit in die Aufnahmefläche (2) hineingezogen, so daß die Aufnahmefläche (2) im Bereich des Schwenkraumes (46) eine verminderte Tiefe (23) gegenüber dem Abstand (11) zwischen dem Scheitelpunkt (18) und dem tiefsten Punkt (41) besitzt. Diese Tiefe (23) ist kleiner als ein Radius (12) des Kugelabschnittes (13. Der dritte Teilbereich (21) spannt zwischen dem ersten Teilbereich (14) und dem zweiten Teilbereich (16) einen dritten Sektorwinkel (24) von etwa 115° auf.

Die Erhöhung (10) besitzt eine dem in die Aufnahmefläche (2) eingesetzten Gelenkkopf (8) zugewandte Gleitfläche (42), die einen Teil der von der Aufnahmefläche (2) ausgebildeten Fläche eines Kugelabschnittes darstellt. Diese Gleitfläche beaufschlagt den in die Aufnahmefläche (2) eingesetzten Gelenkkopf (8).

Der Aufnahmerand (3) bildet eine innere Begrenzung einer aus Teilflächen (5,6,7) zusammengesetzten Begrenzungsfläche (25), wobei der erste Teilbereich (14) die innere Begrenzung der ersten Teilfläche (5), der zweite Teilbereich (16) die innere Begrenzung der zweiten Teilfläche (6) und der dritte Teilbereich (21) die innere Begrenzung der dritten Teilfläche (7) bildet. Die zweite Teilfläche (6) ist gegenüber der ersten Teilfläche (5) verbreitert. Durch die Verbreiterung der zweiten Teilfläche (6) wird an der der zweiten Teilfläche (6) abgewandten Unterseite (26) der Erhöhung (10) ein Ansatz (27) gebildet, mit dem sich die Hüftgelenkpfanne (1) auf dem sie umgebenden Beckenknochen (28) abstützen kann.

Dabei verläuft die zweite Teilfläche (6) in Form eines Anstieges (44) in Richtung auf eine vom Scheitelpunkt (18) gebildete Scheitellinie (40). Der Anstieg (44) erfolgt mit einem Anstiegswinkel (45) von einem Niveau, das von der ersten Teilfläche (5) vorgegeben ist. Der Anstiegswinkel (45) beträgt zwischen 2° und 7°.

Um den Gelenkkopf (8) in die Aufnahmefläche (2) einsetzen zu können, besitzt die angeschrägte Kante im Bereich der dritten Teilfläche (7) eine gebrochene Kante (52), über die der Gelenkkopf (8) in die Aufnahmefläche (2) eingeführt werden kann. Diese gebrochene Kante (52) gestattet eine möglichst weitgehende Anhebung der dritten Teilfläche (7) gegenüber dem eingesetzten Gelenkkopf (8).

Die Hüftgelenkpfanne (1) ist als einteiliger Gelenkkörper (29) ausgebildet. Es ist aber grundsätzlich auch möglich, den Gelenkkörper beispielsweise zur zementlosen Verankerung in Form von zwei oder mehreren ineinander rühenden Schalen auszubilden. Der Gelenkkörper (29) wird nach innen von der Aufnahmefläche (2) und nach außen von der Außenfläche (4), die in einem Abstand zur Aufnahmefläche (2) angeordnet ist, begrenzt.

Die Außenfläche (4) ist ebenfalls schalenförmig, insbesondere kugelförmig ausgebildet. Die Außenfläche (4) weist drei konzentrische Ringnuten (30, 31, 32) auf. Quer zu den Ringnuten (30, 31, 32) sind siebzehn in einem Punkt zusammenlaufende Längsnuten (33) angeordnet. Durch die Längsnuten (33) und die Ringnuten (30, 31, 32) wird eine Vielzahl von ringförmig nebeneinander angeordneten Oberflächensegmenten (34) gebildet, die das Einzementieren der Hüftgelenkpfanne (1) in den Beckenknochen (28) erleichtern. Auf den Oberflächensegmenten (34) eines mittleren Ringes sind auf jedem zweiten Oberflächensegment (34) Abstandshalter (35) angeordnet, die die Hüftgelenkpfanne (1) mit ihrer Außenfläche (4) gegenüber dem benachbarten Beckenknochen (28) auf Abstand halten und für eine gleichmäßige Dicke der zu verwendenden Knochenzementschicht sorgen.

Zum Einsetzen der Hüftgelenkpfanne (1) wird die die Gelenkpfannen (1) aufnehmende Höhlung (37) des Beckenknochens (28) durch Fräsen auf ihre vorgesehene Größe gebracht und die Gelenkpfanne mit Knochenzement (39) in die Höhlung (37) eingesetzt. Die Ausrichtung des Gelenkkörpers (29) bzw. der Hüftgelenkpfanne (1) erfolgt in der Weise, daß der dritte Teilbereich (7) in Richtung eines aus der Aufnahmefläche (2) herausragenden Schaftes angeordnet ist, der in den Femur (47) hineinragt. Auf diese Weise bildet der dritte Teilbereich (7) den Schwenkraum (46) in dem der Schaft (48) Schwenkbewegungen ausführen kann. Bei dieser Ausrichtung der Hüftgelenkpfanne (1) weist die Erhöhung (10) in eine Richtung, in der das in der Hüftgelenkpfanne (1) gelagerte beispielsweise rechte Bein (53) eines Menschen (54) schräg nach hinten weist und dabei eine von einem Standbein (50) in einer Geradeausrichtung aufgespannte gedachte Bewegungsebene (55) in einem spitzen Winkel schneidet.

## Patentansprüche

1. Hüftgelenkpfanne mit einer in eine Höhlung eines sie aufnehmenden Beckenknochens anliegenden Außenfläche und einer einen Gelenkkopf eines künstlichen Hüftgelenks aufnehmenden Aufnahmefläche, die von einem sie begrenzenden Aufnahmerand umgeben ist, dadurch gekennzeichnet, daß der Aufnahmerand (3) an mindestens einer Stelle eine den von der Aufnahmefläche (2) aufgenommenen Gelenkkopf (8) teilweise umfassende Erhöhung (10) aufweist.

2. Hüftgelenkpfanne nach Anspruch 1, dadurch gekennzeichnet, daß die Erhöhung (10) an einer Stelle vorgesehen ist, an der sie den Gelenkkopf (8) in seiner Bewegungsfreiheit am geringsten behindert.

3. Hüftgelenkpfanne nach Anspruch 1 oder 2, dadurch gekennzeichnet, daß die Erhöhung (10) an ihrem vom Aufnahmerand (3) am weitesten aufragenden Scheitelpunkt (18) einen Abstand von einem tiefsten Punkt (41) der Aufnahmefläche (2) aufweist, die den Gelenkkopf (8) weitgehend führend umfaßt.

4. Hüftgelenkpfanne nach einem der Ansprüche 1 bis 3, dadurch gekennzeichnet, daß die Erhöhung (10) vom tiefsten Punkt (41) der Aufnahmefläche (2) zum Scheitelpunkt (18) in einer Richtung aufragt, in der ein das künstliche Hüftgelenk (9) aufnehmender Femur (47) gegenüber einem gestreckten Standbein (50) schräg nach hinten verläuft und dabei eine vom Standbein in eine Geradeausrichtung aufgespannte gedachte Bewegungsebene (55) in einem spitzen Winkel (56) schneidet.

5. Hüftgelenkpfanne nach einem der Ansprüche 1 bis 4, dadurch gekennzeichnet, daß die Aufnahmefläche (2) als ein Kugelabschnitt (13) einer von einer Hohlkugel umschlossenen Innenoberfläche ausgebildet ist, von der eine dem als Kugelkopf ausgebildeten Gelenkkopf (8) zugewandte Gleitfläche (42) der Erhöhung (10) einen Teil bildet.

6. Hüftgelenkpfanne nach einem der Ansprüche 1 bis 5, dadurch gekennzeichnet, daß der Aufnahmerand (3) in einem ersten Teilbereich (14) als Randbereich eines etwa ein horizontaler Ebene (43) verlaufenden Kugelschnittes ausgebildet ist, auf dessen Ebene eine durch den tiefsten Punkt (41) verlaufende Pfannenachse (15) senkrecht steht.

7. Hüftgelenkpfanne nach Anspruch 6, dadurch gekennzeichnet, daß an den ersten Teilbereich (14) des Aufnahmerandes (3) sich ein zweiter, die Erhöhung (10) aufweisender Teilbereich (16) anschließt, der von einem dem ersten Teilbereich (14) benachbarten Anlaufende (17) des zweiten Teilbereiches (16) bis zum Scheitelpunkt (18) zu der Erhöhung (10) hin ansteigt.

8. Hüftgelenkpfanne nach Anspruch 7, dadurch gekennzeichnet, daß der zweite Teilbereich (16) zwischen seinem Anlaufende (17) und dem Scheitelpunkt (18) gegenüber der Pfannenachse (15) einen zweiten Sektorwinkel (19) zwischen 45° und etwa 135° aufspannt.

9. Hüftgelenkpfanne nach Anspruch 8, dadurch gekennzeichnet, daß der zweite Sektorwinkel (19) des zweiten Teilbereiches (16) etwa 110° beträgt.

10. Hüftgelenkpfanne nach einem der Ansprüche 7 bis 9, dadurch gekennzeichnet, daß der zweite Teilbereich (16) in Richtung auf den Scheitelpunkt (18) einen weitgehend konstanten Anstieg (44) aufweist.

11. Hüftgelenkpfanne nach Anspruch 10, dadurch gekennzeichnet, daß der Anstieg (44) einen Anstiegswinkel (45) von etwa 2° bis 7° aufweist.

12. Hüftgelenkpfanne nach einem der Ansprüche 6 bis 11, dadurch gekennzeichnet, daß zwischen einem dem Anlaufende (17) des zweiten Teilbereiches (16) abgewandten ersten Ende (20) des ersten Teilbereiches (14) und dem Scheitelpunkt (18) der Erhöhung (10) des zweiten Teilbereiches (16) ein dritter Teilbereich (21) angeordnet ist, in dem der Aufnahmerand (3) in Richtung auf den tiefsten Punkt (41) zur Freigabe eines Schwenkraumes (46) abgesenkt ist, innerhalb dessen einer im Femur (47) befestigbarer Schaft (48) des Kugelkopfes schwenkbar lagerbar ist.

13. Hüftgelenkpfanne nach Anspruch 12, dadurch gekennzeichnet, daß der Schwenkraum (46) unmittelbar in Richtung auf das erste Ende (20) vom Scheitelpunkt (18) abfällt und im Bereich des Schwenkraumes (46) der Aufnahmerand (3) in eine Ebene (49) angeschrägt ist, die im spitzen Winkel (50) zu einer sich durch die Pfannenachse (15) erstreckenden lotrechten Ebene (51) verläuft.

14. Hüftgelenkpfanne nach Anspruch 13, dadurch gekennzeichnet, daß der spitze Winkel (50) im Bereich zwischen 30° und 60° liegt.

15. Hüftgelenkpfanne nach Anspruch 14, dadurch gekennzeichnet, daß der spitze Winkel (50) 40° beträgt.

16. Hüftgelenkpfanne nach einem der Ansprüche 13 bis 15, dadurch gekennzeichnet, daß der Aufnahmerand (3) im Schwenkraum (46) eine den Kugelkopf formschlüssig führende Tiefe (23) gegenüber dem tiefsten Punkt (41) und eine das Einsetzen des Kugelkopfes in die Aufnahmefläche (2) erlaubende gebrochene Kante (52) aufweist.

17. Hüftgelenkpfanne nach einem der Ansprüche 13 bis 16, dadurch gekennzeichnet, daß der dritte Teilbereich (21) zwischen dem ersten Teilbereich (14) und zweiten Teilbereich (16) gegenüber der Pfannenachse (15) einen dritten Sektorwinkel (24) zwischen etwa 90° und etwa 180° aufspannt.

18. Hüftgelenkpfanne nach Anspruch 17, dadurch gekennzeichnet, daß der dritte Teilbereich (21) einen dritten Sektorwinkel (24) von etwa 115° aufspannt.

19. Hüftgelenkpfanne nach einem der Ansprüche 6 bis 18, dadurch gekennzeichnet, daß der Aufnahmerand (3) als eine der Pfannenachse (15) zugewandte innere Begrenzung einer aus Teilflächen (5,6,7) zusammengesetzten Begrenzungsfläche (25) ausgebildet ist.

20. Hüftgelenkpfanne nach Anspruch 19, dadurch gekennzeichnet, daß der erste Teilbereich (14) des Aufnahmerandes (3) die innere Begrenzung der ersten Teilfläche (5), der zweite Teilbereich (16) die innere Begrenzung der zweiten Teilfläche (6) und der dritte Teilbereich (21) die innere Begrenzung der dritten Teilfläche (7) bildet.

21. Hüftgelenkpfanne nach Anspruch 20, dadurch gekennzeichnet, daß die zweite Teilfläche (6) gegenüber der ersten Teilfläche (5) verbreitert ist.

22. Hüftgelenkpfanne nach einem der Ansprüche 1 bis 21, dadurch gekennzeichnet, daß sie als ein einheitlicher Gelenkkörper (29) ausgebildet ist.

23. Hüftgelenkpfanne nach einem der Ansprüche 1 bis 21, dadurch gekennzeichnet, daß sie als ein mehrteiliger Gelenkkörper (29) ausgebildet ist.

24. Hüftgelenkpfanne nach einem der Ansprüche 1 bis 23, dadurch gekennzeichnet, daß die Außenfläche (4) etwa kugelförmig ausgebildet ist.

25. Hüftgelenkpfanne nach Anspruch 24, dadurch gekennzeichnet, daß die Außenfläche (4) eine Mehrzahl von etwa konzentrisch zur Pfannenachse (15) verlaufende Ringnuten (30, 31, 32) aufweist.

26. Hüftgelenkpfanne nach Anspruch 25, dadurch gekennzeichnet, daß die Außenfläche (4) drei konzentrische Ringnuten (30, 31, 32) aufweist.

27. Hüftgelenkpfanne nach Anspruch 25 oder 26, dadurch gekennzeichnet, daß die Außenfläche (4) eine Mehrzahl von quer zu den Ringnuten (30, 31, 32) verlaufenden Längsnuten (33) aufweist.

28. Hüftgelenkpfanne nach Anspruch 27, dadurch gekennzeichnet, daß die Außenfläche (4) etwa 17 Längsnuten (33) aufweist.

29. Hüftgelenkpfanne nach Anspruch 1 bis 28, dadurch gekennzeichnet, daß die Außenfläche (4) eine Mehrzahl von etwa konzentrisch zur Pfannenachse (15) angeordneten Abstandshaltern (35) aufweist.

30. Hüftgelenkpfanne nach einem der Ansprüche 1 bis 29, dadurch gekennzeichnet, daß der Gelenkkörper (29) aus Kunststoff ausgebildet ist.

31. Hüftgelenkpfanne nach Anspruch 30, dadurch gekennzeichnet, daß der Gelenkkörper (29) aus einem Polyäthylen besteht.
